# EUROPEAN PATENT APPLICATION

(11) **EP 0 876 821 A2**
(43) Date of publication of application: **11.11.1998**
(21) Application number: 98303324.2
(22) Date of filing: 28.04.1998
(51) Int. Cl.: A61L 27/00

(54) **A process for improving start up and steady rate friction of soft/compliant polyurethanes**

(30) Priority: 02.05.1997 GB 9709071
(71) Applicant: HOWMEDICA INTERNATIONAL INC., Shannon Co. Clare (IE)
(72) Inventor: Lawes, Peter, Maidenhead, Berkshire, SL6 4DB (GB); Smith, Nigel, Dr., Wokingham, Berkshire, RG11 1LH (GB)
(74) Representative: Bridge-Butler, Alan James

(57) **Abstract**

A process for improving start up and steady rate friction of soft/compliant polyurethanes in an aqueous lubricant which includes treating a polyurethane element by applying a layer of diamond-like carbon (dlc) coating.

## Description

This invention relates to a process for improving start up and steady rate friction of soft/compliant polyurethanes and to polyurethane elements which have been treated by the process. An important potential application for such treated material is in compliant layer bearings for example those used in artificial joint replacements, although it also has other applications.

Typically these artificial joint replacement bearings are relatively recent and rely on the use of soft/compliant polyurethanes (or other elastomeric materials) to improve the lubrication mechanism compared with conventional artificial joints. The concept of using a compliant polyurethane layer on the bearing surface of an artificial joint replacement is inspired by the natural synovial joint which has such a compliant layer, the articular cartilage. A combination of lubrication mechanisms have been proposed for synovial joints which result in fluid film lubrication, where the joint surfaces are completely separated by a thin film of lubricant. Currently most artificial joints are based upon metal on ultra high molecular weight polyethylene (UHMWPE), ceramic on UHMWPE or metal on metal material couples. These bearings are far less compliant than the natural joint and hence operate in a mixed lubrication regime, with partial contact of the two bearing surfaces. This leads to higher friction, and wear of the bearing surfaces of conventional joints. In contrast, compliant bearings operate with fluid film lubrication and extremely low friction, and hence potentially negligible wear and a long implant life.

The design parameters for the construction of compliant layer bearings are known. Polyurethane elastomers have been the materials of choice for these bearings. Under conditions of cyclic loading and motion typical of the major load bearing joints in the human body there is much experimental evidence that this type of bearing will operate with extremely low friction, typical of fluid film lubrication. In contrast, it has been reported by Caravia et al that the use of compliant polyurethane layers in this application can result in unacceptably high friction values under conditions which combine heavy loading and low sliding velocities, i.e. at the onset of motion (start up friction).

The purpose of this invention is to address the important area of improving start up friction whilst maintaining effective fluid film lubrication during normal cyclic loading and motion.

The following definitions are used herein
- coefficient of friction (µ):: the ratio of tangential frictional force to the normal load for a plane surface.
- friction factor (**f**):: the ratio of the product of frictional torque (**T**) and cup radius to the normal load for the cupped geometry considered in this study, during steady state motion.
- frictional torque (**T**):: the torque required to resist rotation of the compliant layered cup about an axis perpendicular to the axis of loading, under the normal load and motion conditions.
- start up friction factor (**f**_{**s**}):: friction factor (**f**) at the onset of motion.

According to the present invention a process for improving start up and steady rate friction of soft/compliant polyurethanes in an aqueous lubricant which includes treating a polyurethane element by applying a layer of diamond-like carbon (dlc) coating.

Preferably the process includes subjecting the polyurethane element to a stream of ions of a suitably inert gas in a vacuum chamber and then using a fast atom bombardment (FAB) source, or a similar low temperature method such as radio frequency discharge, direct or dual beam deposition, magnetron sputtering or cyclotron resonance chemical vapour deposition, to yield a coherent, amorphous diamond-like carbon layer adherent to the prepared polyurethane surface.

The inert gas can be argon or helium and a stream of hydrocarbon gas, such as acetylene can be used to bombard the prepared polyurethane surface.

The invention also includes a polyurethane element which has been treated by the process set forth.

The element can be or form part of a surgical or medical device which contacts body tissue and fluids, for example a prosthetic device, a stent, a catheter or an angio plastic balloon.

The devices referred to above all operate with an aqueous solution in the body or with an aqueous lubricant for example synovial fluid.

As mentioned above material treated by the process can be used as a bearing surface and thus a prosthetic device according to the invention can have a bearing surface at least part of which is formed from a polyurethane element which has been treated by the method set forth above.

The prosthetic device can have a first bearing surface formed from the treated material and a second co-operating surface formed form, for example, metal. The metal can be cobalt chrome steel or similar metal or alloy used in implantable medical devices and it can also be provided with a diamond-like carbon (dlc) coating.

When applied to, for example, a hip joint the first treated surface can be provided on an acetabular cup and the second co-operating surface can be the co-operating ball head of an implant.

The invention can also be applied to, for example, tibial bearings suitable for total knee replacements.

The invention can be performed in many ways and some embodiments will now be described by way of example and with reference to the accompanying drawings in which :
Figure 1 is a diagram showing start up friction factor against preload time;
Figure 2 is a graph showing start up friction for an unconditioned cup with CoCr head combination;
Figure 3 is a graph showing start up friction for an unconditioned cup with a dlc head combination;
Figure 4 is a graph showing start up friction for a conditioned cup with dlc head combination;

The present invention addresses the issue of improving start up friction of compliant bearings designed for orthopaedic applications and other uses whilst maintaining effective fluid film lubrication mechanism under normal cyclic loading and motion.

The evaluation of the frictional properties of compliant layers referred to herein describes the properties of both "dry" contacts, and those lubricated with a low viscosity lubricant, i.e. pathological pseudo synovial fluid. The friction properties of the bearings have been measured on a machine which simulates the loads and motions experienced at the hip joint both at the start and during steady state motion.

In order to evaluate the invention the process was applied to polyurethane acetabular cups the object being to yield a strong bond between a polymer substrate, for example, a soft polyurethane, and a rigid smooth layered film i.e. diamond like carbon. The method being to subject the polymer substrate to a stream of ions of a suitable inert gas in a vacuum chamber. The aforementioned ions having sufficient energy and force to modify the surface characteristics of the polymer substrate. Afterwards, to use a fast atom bombardment (FAB) source to yield a coherent, amorphous diamond layer adherent to the prepared polymer surface.

The method is described in detail below as applied to flat surfaces.

Injection moulded test pieces (150 x 30 x 3 mm) were cleaned prior to being introduced to the coating chamber by ultrasonication in detergent followed by a rinse in alcohol i.e. methanol. The substrate was then treated for a range of time period (5, 10, 15, 20 minutes) under a stream of argon or helium. The operating conditions for the pre-treatment are summarised in Table 1.

**Table 1**

| Time (min) | Current (mA) | Voltage (kV) | Ar or He flow | Pressure (mB) |
|---|---|---|---|---|
| 0 | 400 | 0.6 | 20.4 | 3 x 10-3 |
| 5 | 400 | 0.6 | 20.4 | 3 x 10-3 |
| 10 | 400 | 0.7 | 20.3 | 3 x 10-3 |
| 15 | 400 | 0.7 | 20.3 | 3 x 10-3 |
| 20 | 400 | 0.7 | 20.3 | 3 x 10-3 |

The pre-treated polymer substrates were then coated with an amorphous diamond layer to obtain coating thickness between 0.07 and 0.8 microns using a stream of acetylene. The operating conditions for the coating deposition are summarised in Table 2.

**Table 2**

| Time (hrs) | Current (mA) | Voltage (kV) | gas flow | Pressure (mB) |
|---|---|---|---|---|
| 4 | 400 | 1 | 20.4 | 3 x 10-3 |
| 6 | 400 | 1 | 20.4 | 3 x 10-3 |
| 8 | 400 | 1 | 20.3 | 3 x 10-3 |
| 16 | 400 | 1 | 20.3 | 3 x 10-3 |
| 6 | 300 | 1 | 23.6 | 5 x 10-3 |
| 6 | 500 | 1 | 23.6 | 4 x 10-3 |
| 6 | 600 | 1.2 | 24.2 | 3 x 10-3 |

The diamond-like coatings were characterised by surface measurements, micro-hardness and adhesion test.

The optimum coatings systems was applied to a set of compliant layer cups to assess, both friction measurements and wear properties. The hip cups were assessed as described below, and this has demonstrated a consistent improvement in start-up frictional performance compared to an unconditioned cup.

A conventional 32 mm CoCr femoral head (manufactured by Howmedica) was also treated using a commercial diamond-like carbon process i.e. plasma assisted chemical vapour deposition (PA CVD) to yield a coherent dlc coating over the articulating surface of the femoral head. The femoral head was used in the start-up friction experiments described below, and as before provided an improvement in the start-up friction in all cases.

Frictional measurements (dry) were made with a simulator to measure the frictional torque developed in a hip joint under cyclic loads and motions typical of physiological conditions. The machine used is similar to that described by Unsworth, A. Dowson, D. Wright, V. (1974a) (The frictional behaviour of human synovial joints - Part 1: Natural joints. Trans. ASME: J. Lub. Tech., **74-Lub-38**). This apparatus is housed at Durham University, England. The apparatus consists of three main systems; which applied the joint load, drove the motion and measured the resulting frictional torque. The test hip joint was mounted with its centre of rotation incident with the centre of rotation of the simulator. The components were anatomically inverted, that is with the femoral head above the acetabular cup. A load of 1,000 N was used to protect the piezo electric transducer used in the simulator. Untreated (unmodified as manufactured) acetabular cups were used and the result is shown in Table 3 below.

**Table 3 .**

| Dry friction factors of common counterfaces with candidate polyurethanes compliant layers. | | | | |
|---|---|---|---|---|
| Polyurethane layer | CoCr Head | | Ceramic Head | |
| | mean | s.d. | mean | s.d. |
| CSIRO | 1.07 | 0.12 | 0.83 | 0.17 |
| Corethane | 1.01 | 0.18 | 1.02 | 0.13 |
| Chronoflex | 0.98 | 0.08 | 0.95 | 0.12 |
| Tecoflex | 0.88 | 0.13 | 0.94 | 0.14 |
| Tecothane | 0.77 | 0.30 | 0.93 | 0.17 |

The results showed that with typical polyurethane layers the dry friction factor values were typically 0.8 to 1.1. A friction factor of 1.0 would result in a frictional torque of approximately 30 Nm for an applied load of 2 kN acting to rotate a 32 mm diameter acetabular cup. This approaches the value of 100 Nm, reported by Anderson et al. as the static torque required to dislodge a well fixed cemented prosthesis.

Consequently, should the bearings run dry or intimate contact between the bearing surfaces occur, the level of friction generated under these conditions with untreated compliant layer bearings would be sufficiently high to risk accelerated loosening and could result in early failure.

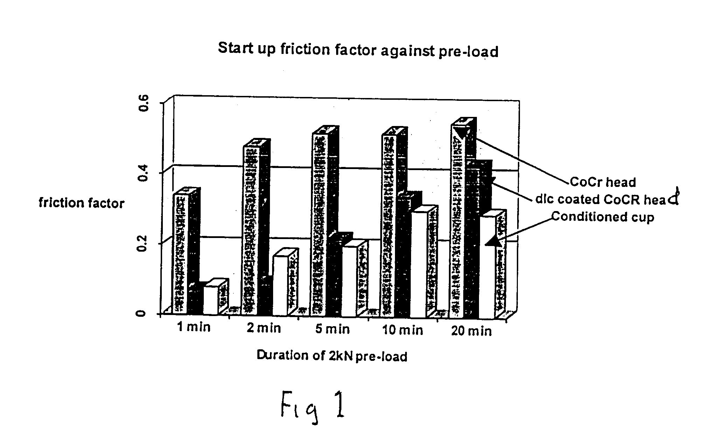

Frictional measurements for lubricated start-up friction were carried out on unconditioned cups and cups treated according to the invention. During operation of the Durham hip function simulator, a preload of 2 kN was applied to mimic the effect of standing and loading an acetabular bearing without any flexion-extension motion. This was achieved by operating the simulator in the "reverse" loading mode. The "reverse" mode cycle started with a high load, and hence a delay in starting the motion enabled the test cups to experience a fixed preload of 2 kN for 1, 2, 5, 10 and 20 mins. The frictional torque during the first two cycles was measured, using water as the lubricant, for the head/cup combinations summarised in Table 4, and the results plotted against preload time in Figure 1.

**Table 4 .**

| Start up friction factors. | | | | | | |
|---|---|---|---|---|---|---|
| Cup | Head | Start-up factor at different duration's of 2 kN preload | | | | |
| | | 1 min | 2 min | 5 min | 10 min | 20 min |
| Unconditioned cup | CoCr head | 0.34 | 0.48 | 0.52 | 0.52 | 0.55 |
| Unconditioned cup | dlc coated CoCr head | 0.07 | 0.09 | 0.22 | 0.34 | 0.43 |
| dlc coated cup | dlc coated CoCr head | 0.13 | 0.22 | 0.25 | 0.21 | 0.22 |

Conditioning was conducted as described above.

In general the results show that as relative motion starts the frictional torque is high, but that this quickly reduces within one cycle, to the extremely low values expected from compliant layer bearings. There are two characteristics which have been used to differentiate between the material couples, that is the initial friction factors given in Table 4 and Figure 1, and the rate at which the frictional torque decreases during the period of initial motion.

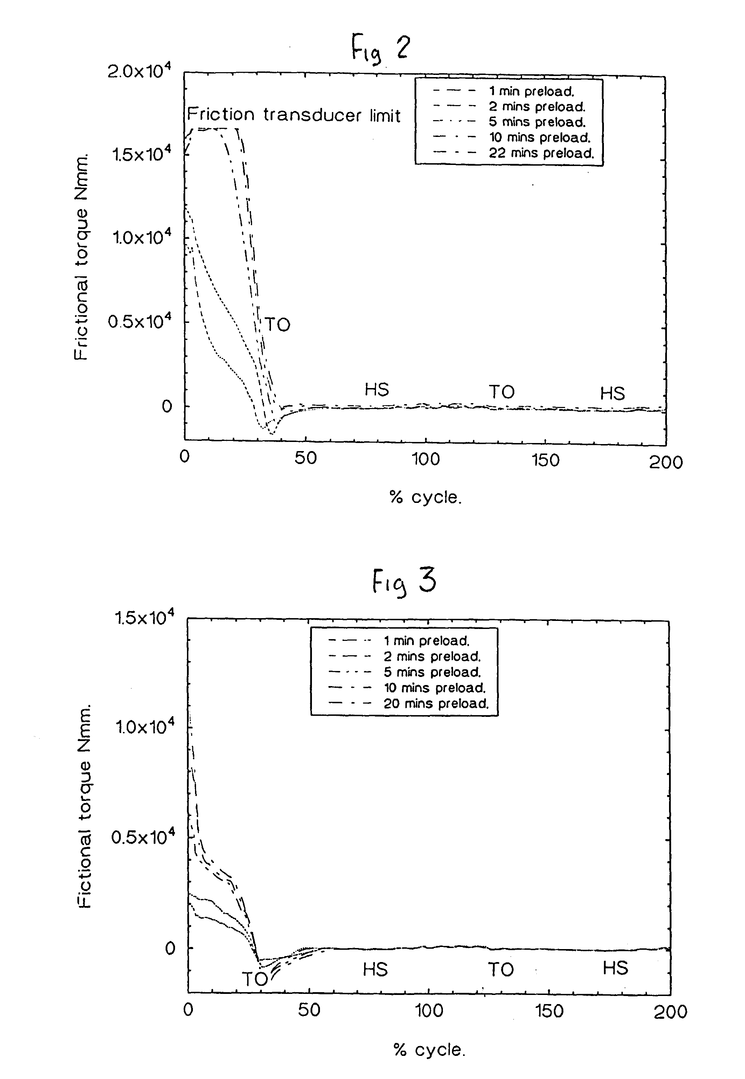

The unconditioned cup and cobalt chrome steel head develops very high frictional torque which remains high throughout the period of loading, Figure 2. If a dlc coated head is used with the same unconditioned cup then the initial frictional torque is lower (especially under shorter preload duration) Figure 3, and it reduces still further as fluid is entrained during the motion cycle.

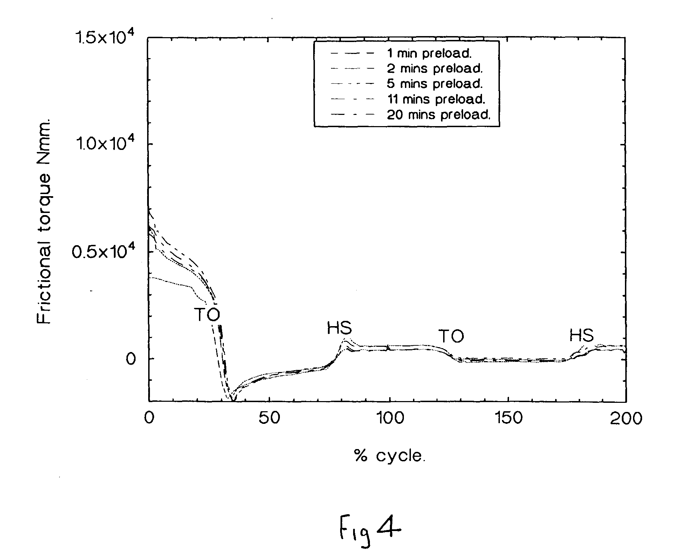

The conditioned cup,according to the invention as shown in Figure 4, develops a relatively moderate frictional torque or start up with less dependence on preload times, although the torque reduces slowly during the period of loading.

Caravia et al. assessed start-up friction developed between a thin polyurethane layer and several types of indenter using a pin on plate friction rig. A constant contact stress of 2 MPa and a sliding velocity of 8 mms-1 was used. The indenter was loaded for between 5 s and 400 s and the peak friction measured using water as a lubricant. In contrast, this simulator experiment used a dynamically applied load with a maximum contact stress of 7.3 MPa, a sinusoidal sliding velocity of maximum 34 mms⁻¹, and a preload of 2 kN applied for between 60 s and 1200 s.

Caravia et al. showed that the start-up friction increased with increasing preload time up to 80 s, after which they suggested the squeeze film action reached equilibrium and the friction increased no further. They reported higher values of start-up friction than this study, i.e. coefficients of friction between 0.6 and 1.1 for similar modulus material at 160 s preload. They also suggested that the surface energy of the bearing counter face could also be an important factor.

The experimental results of conditioned elements according to the present invention can be summarised:
The dlc coated cup gives a consistent improvement in start-up largely independent of preload time.

This work suggests that through the application of this invention, there is :
1) A significant reduction in start up friction.
2) A reduction in the effect of static preload on the frictional torque in the initial phase of motion.

These important results are critical to the long term performance of these bearings, since in contrast to the results presented by Caravia et al. which suggests start up friction may limit the life of such devices, these inventions significantly reduce this potential problem.

It was observed during the experiments described above that the effects of treating the compliant bearing acetabular cups improved both the start up friction and the steady rate friction characteristics. The frictional changes were related to an affect of surface re-organisation that occurs both within the bulk and at the surface of these polyurethane materials. These changes have been examined by the techniques of surface analysis such as ATR-fourier transform infra-red spectroscopy (ATR-FTIR), dynamic contact angle (DCA) and water uptake studies. Thus the process to promote these beneficial changes was optimised.

These significant improvements in the start up and steady state friction have been achieved using the processes of the invention, either alone or in combination. The application of these developments is primarily targeted at bearing systems for joint or surface replacement involving, but not limited to the hip, knee, elbow, shoulder and ankle. The major purpose is to apply for example, a layer of diamond like carbon (dlc) coating to modify the surface chemistry of linear polyurethane's, or cobalt chrome steel or similar metal or alloy to attain a hydrophobic surface that has a high contact angle. This method of surface modification can not only be applied to compliant layer joints but also other medical devices that contact body tissues and fluids i.e. stents, catheters, angioplasty balloons etc.

A further application is associated with reducing friction in mechanical systems where sliding contacts are involved i.e. bushes (cylindrical or flat) that operate using predominantly aqueous lubricants, for example, in tibial bearings suitable for total knee replacements.

## Claims

1. A process for improving start up and steady rate friction of soft/compliant polyurethanes in an aqueous lubricant which includes treating a polyurethane element by applying a layer of diamond-like carbon (dlc) coating.

2. which includes subjecting the polyurethane element to a stream of ions of a suitably inert gas in a vacuum chamber and then a low temperature deposit process to yield a coherent, amorphous diamond-like carbon layer adherent to the prepared polyurethane surface.

3. A process as claimed in claim 2 in which the lower temperature deposit process includes a fast atom bombardment (FAB) source, a radio frequency discharge, direct or dual beam deposition, magnetron sputtering or cyclotron resonance chemical vapour deposition.

4. A process as claimed in claim 1, claim 2 or claim 3 in which the inert gas is argon or helium.

5. A process as claimed in any one of preceding claims 2 to 4 in which a stream of hydrocarbon gas is used to bombard the prepared polyurethane surface.

6. A process as claimed in claim 5 in which the hydrocarbon gas is acetylene.

7. A polyurethane element which has been treated by the process set forth in any one of the preceding claims 1 to 6.

8. A polyurethane element as claimed in claim 7 which can be or forms part of a surgical or medical device which contacts body tissues and fluids when in use.

9. A polyurethane element as claimed in claim 8 in which the surgical or medical device is a prosthetic device, a stent, a catheter or an angio plastic balloon.

10. A prosthetic device having a bearing surface at least part of which is formed from a polyurethane element which has been treated by the method set forth in any one of preceding claims 1 to 6.

11. A prosthetic device as claimed in claim 8 which has a first bearing surface formed from said treated material and a second co-operating surface is formed from metal.

12. A prosthetic device as claimed in claim 11 in which the metal is cobalt chrome steel.

13. A prosthetic device as claimed in claim 9 in which the second co-operating surface is also provided with a diamond-like carbon (dlc) coating.

14. A prosthetic device as claimed in any one of preceding claims 10 to 13 in which said first treated surface is provided on an acetabular cup and the second co-operating surface is a co-operating ball head of an implant.
